Europäisches Patentamt

(19) European Patent Office

Office européen des brevets

(11) EP 0 941 727 A1

(12) EUROPEAN PATENT APPLICATION

(43) Date of publication:
15.09.1999 Bulletin 1999/37

(51) Int. Cl.$^6$: A61F 13/15

(21) Application number: 98104437.3

(22) Date of filing: 12.03.1998

(84) Designated Contracting States:
AT BE CH DE DK ES FI FR GB GR IE IT LI LU MC
NL PT SE
Designated Extension States:
AL LT LV MK RO SI

(71) Applicant:
THE PROCTER & GAMBLE COMPANY
Cincinnati, Ohio 45202 (US)

(72) Inventor:
Schmitz, Christoph Johann
53881 Euskirchen-Stotzheim (DE)

(74) Representative:
Kohol, Sonia et al
Procter & Gamble
European Service GmbH
Sulzbacher Strasse 40-50
65823 Schwalbach am Taunus (DE)

(54) Disposable absorbent undergarment having a continuous elasticized waistband

(57) A disposable garment (10) having a pant body (12) defining a waist opening (24) and a pair of leg openings (26). The disposable garment body (12) has a front section (14), a back section (16), and a crotch section (18). The pant (10) includes a continuous waistborder (30) which includes a front waistborder section (42) in the front section (14) and a back waistborder section (46) in the back section (16). The front waistborder section (42) has a length dimension and the back waistborder section (46) has a length dimension. An elastic waistband which includes a front waistband portion (64) joined to the front waistborder section (42) and a back waistband portion (66) joined to the back waistborder section (46). The front waistband portion (64) has a length dimension and the back waistband portion (66) has a length dimension. At least one of the elastic waistband portions (64,66) has a length dimension greater than the length dimension of the respective waistborder section (42,46) to which the elastic waistband portion (64,66) is joined.

FIG. 2

## Description

### BACKGROUND OF THE INVENTION

The present invention relates to a disposable absorbent garment, and more particularly to a disposable absorbent garment and a continuous, elasticized waistband joined about an opening thereof for improving fit and comfort.

In the last several years, disposable absorbent garments have become available for use by children in the potty-training stage, and have proved to be extremely popular with mothers and caretakers. A specific example is a training pant comprising a bodyside liner, an outer cover, an absorbent medium between the liner and the outer cover, and side seams that bond portions of the side edges of the pant together to form a waist opening and leg openings.

One style of training pant has elastic side panels that fit against the hips of the child, and discrete front and back elastic waist strips adjacent the waist opening. The waist strips generally are spaced from the elastic side panels.

Although these discrete front and back elastic waist strips may provide some gasketing at the front and back of the pant, they are not entirely effective in providing the desired gasketing and fit at the waist resulting in satisfactory waste containment.

U.S. Pat. No. 5, 545, 158 discloses a pant having an elasticized waistband. The problem with the waistband of 5, 545, 158 is that it stops short of completing a continuous elasticized loop as portions of the waistband which are in the bonded side seam will be rendered inelastic.

The present invention solves the problem of 5, 545, 158 by providing a waistband having a dimension greater than that of the portion of the pant to which it is joined permitting the waistband to be overlapped ensuring complete elasticity about the waist opening of the pant.

### SUMMARY OF THE INVENTION

The present invention provides a disposable garment comprising a pant body defining a waist opening and a pair of leg openings. The pant has a front section, a back section, and a crotch section. The pant includes a continuous waistborder comprising a front waistborder section in the front section and a back waistborder section in the back section. The front waistborder section has a length dimension and the back waistborder section has a length dimension. An elastic waistband comprising a front waistband portion joined to the front waistborder section and a back waistband portion joined to the back waistborder section. The front waistband portion has a length dimension and the back waistband portion has a length dimension. At least one of the elastic waistband portions has a length dimension greater than the length dimension of the respective waistborder section to which the elastic waistband portion is joined.

In one embodiment the front waistband portion has a length dimension greater than the length dimension of the front waistborder section.

In another embodiment, the back waistband portion has a length dimension greater than the length dimension of the back waistborder section.

Alternatively, the back waistband portion has a length dimension greater than the length of the back waistborder section and the front waistband portion has a length dimension greater than the length dimension of the front waistborder section.

### BRIEF DESCRIPTION OF THE DRAWINGS

The above-mentioned and other features of the present invention and the manner of attaining them will become more apparent, and the invention itself will be better understood by reference to the following description of the invention, taken in conjunction with the accompanying drawings, wherein:

FIG. 1 illustrates a front perspective view of one type of a disposable elasticized, absorbent garment incorporating the principles of the present invention;

FIG 2 illustrates a top plan view of the garment in FIG. 1 in a partially disassembled, extended flat state;

FIG. 3 illustrates an exploded, perspective view of the garment in FIG. 1 in a partially disassembled, extended flat state;

FIG. 4 illustrates a top plan view of an alternative embodiment of a pant of the present invention;

FIG. 5 illustrates a top plan view of another embodiment of a pant of the present invention; and

FIG. 6 is a perspective illustration of the waistband of the present invention.

### DEFINITIONS

Each of the following terms used herein include the following meaning:

"Composite elastic material" or "composite elastic web" means a multi-layered material having at least one elastic layer joined to at least one gatherable layer at least at two locations wherein the gatherable layer is gathered between the locations where it is joined to the elastic layer. A composite elastic material may be stretched to the extent that the non-elastic material gathered between the bond locations allows the elastic material to extend.

"Continuous" means that the described structure is a closed-loop structure. The continuous structure may be unitary, i.e., a one-piece structure, or may be made up of individual elements suitably joined together to form a closed-loop.

"Disposable" means that the described garment is designed to be used until soiled, either by urination, defecation, or otherwise, and then discarded, rather than being washed and used again.

"Elastic", "elasticity", "elasticized", or the like, refers to a material or composite material that tends to recover its original size and shape after removal of the force causing the deformation (expressed in %).

"Elongation" means the ratio of the extension of a material to the length of the material prior to the extension (expressed in %), as represented by the following:

$$\frac{\text{extended length - original length}}{\text{original length}} \times 100$$

"Extension" means the change in length of a material due to stretching (expressed in units of length).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Operatively elastically joined" describes the joining of an elastic member to a non-elastic member such that the two joined members exhibit elasticity.

"Pant body" refers to a garment that has a waist opening and a pair of leg openings, similar to shorts, swim wear, or the like. The described garment may or may not have a manually tearable side seam.

"Recover", "recovering", or variations thereof, refers to a contraction of an extended material upon termination or removal of a biasing force, or upon suitably treating the material after it has been temporarily inhibited.

"Temporarily inhibit" means to delay the total recovery of an extended elastic layer (or substrate) or composite elastic material. The delay may be imparted by compressing the extended elastic layer, or by compressing the composite elastic material so that the elastic and gatherable layers are temporarily joined. Partial recovery of a temporarily inhibited elastic layer or composite elastic material may occur immediately after the force is removed, but total recovery of such a temporarily inhibited elastic layer or composite elastic material will require more time than the total recovery of the same material which has not been temporarily inhibited. For example, total recovery of an extended elastic layer or composite elastic material that has not been temporarily inhibited may be instantaneous, whereas the total recovery of a temporarily inhibited elastic layer or composite elastic material may take, for example, from about 5 to about 60 seconds.

"Total recovery", or variations thereof, refers to a material recovering to generally within about 20 percent of its relaxed, preextended dimension.

DETAILED DESCRIPTION

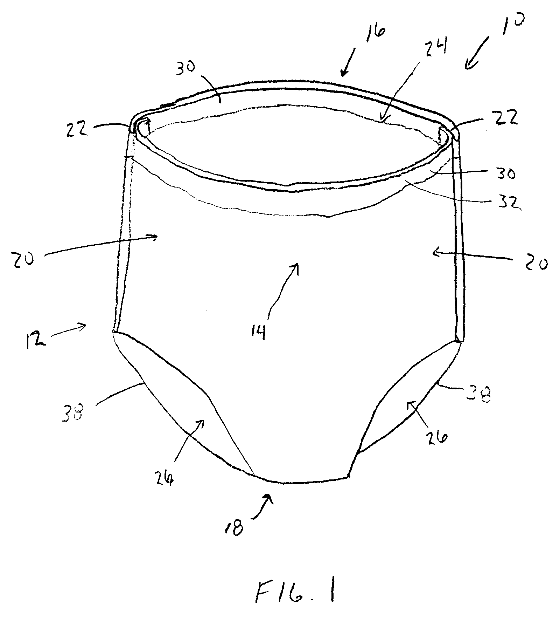

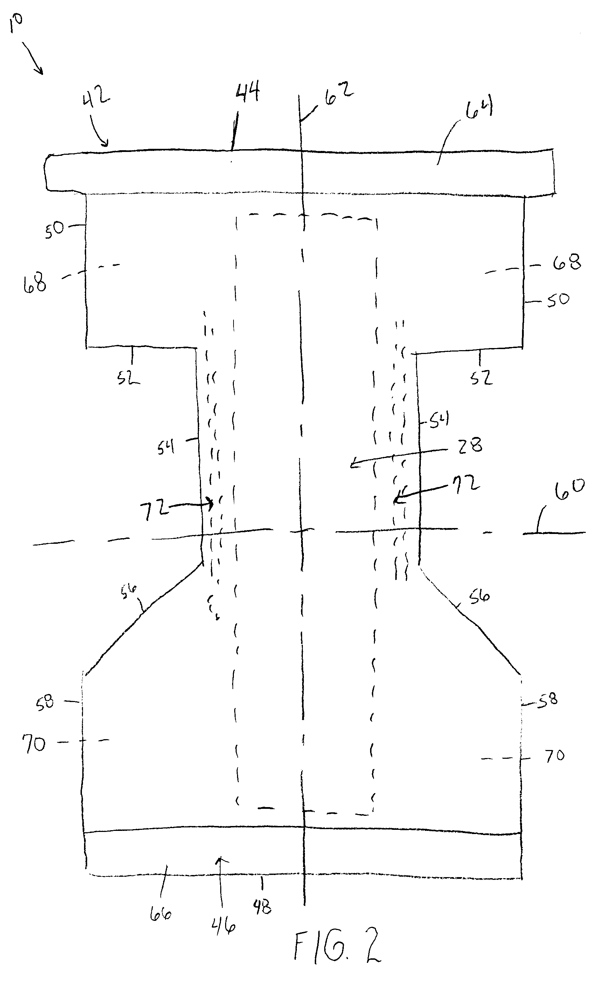

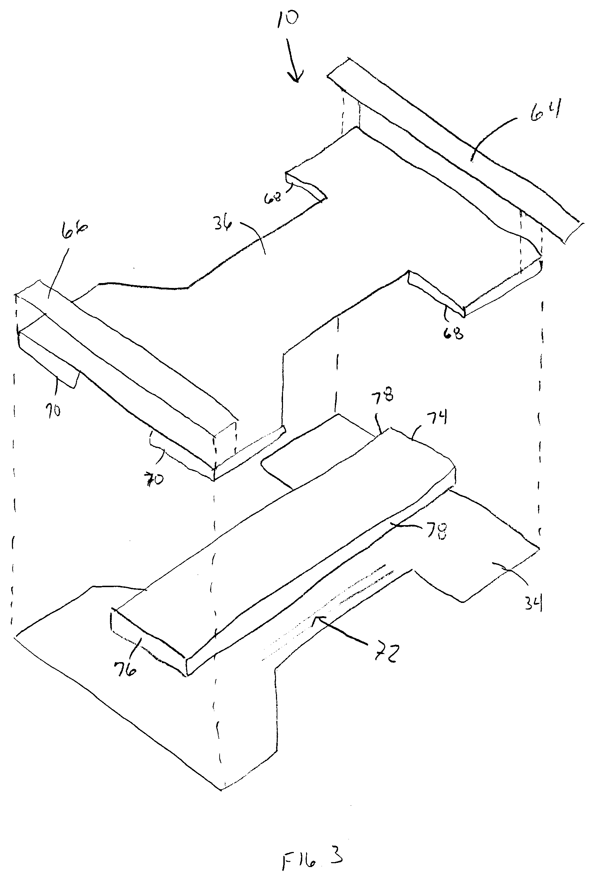

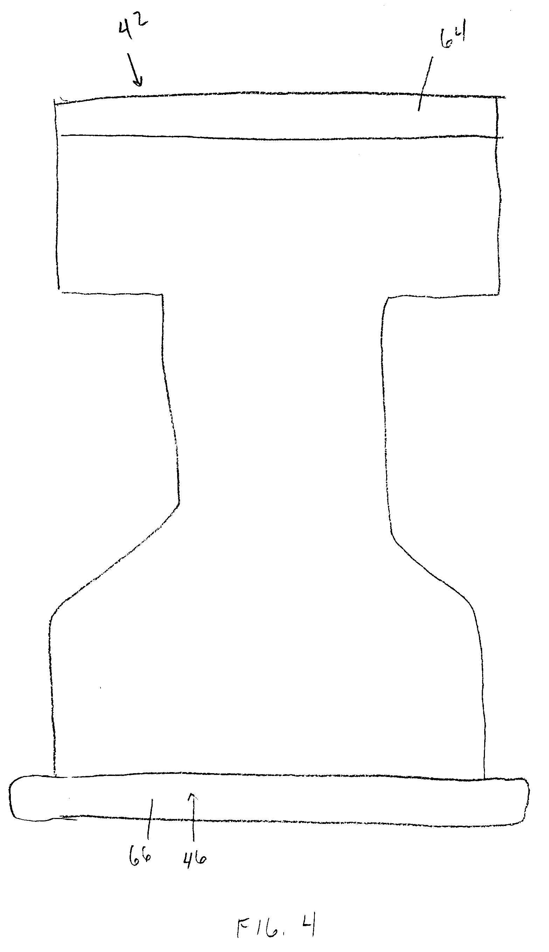

Referring to FIGS. 1-3 there is illustrated disposable absorbent garment 10 in the form of a child's training pant. Although garment 10 is illustrated and will be described as a training pant, it can be other types of absorbent garments or articles, such as baby diapers, adult incontinence products, or the like.

Disposable absorbent garment 10 includes a pant body 12 comprising a front section 14, a back section 16, a crotch section 18, elastic side sections 20, seams 22, a waist opening 24, and a pair of leg openings 26. Each elastic side section 20 includes a front elastic side member 68 and a back elastic side member 70, which are preferably joined together at a respective seam 22. Garment 10 further includes a continuous waistborder 30, continuous legborders 38, and a continuous waistband 32. Continuous waistband 32 comprises front waistband portion 64 and back waistband portion 66. Continuous waistborder 30 comprises front waistborder section 42 having front edge 44 and back waistborder section 46 having back edge 48. An absorbent structure 28 is suitably incorporated in garment 10 at least at crotch section 18 thereof.

Pant body 12 includes a topsheet 34 and a backsheet 36, which are desirably coincident with one another, although not a requirement of the present invention. Backsheet 36 includes a pair of front outer edges 50, front inner edges 52, innermost edges 54, back sloping edges 56, and back outer edges 58.

Front waistband portion 64 is suitably joined to front waistborder section 42, and back waistband portion 66 is suitably joined to back waistborder section 46. Front and back waistborder sections 42, 46 desirably have respective lengths dimensions.

At least one of the waistband portions, either front waistband portion 64 or back waistband portion 66, has a length dimension greater than the length dimension of the waistborder section to which it is joined. To ensure that the waistbands overlap to create complete elasticity about the waist opening, the total length dimension of the front and back waistband portions must be greater than the total length dimension of the front and back waistborder sections.

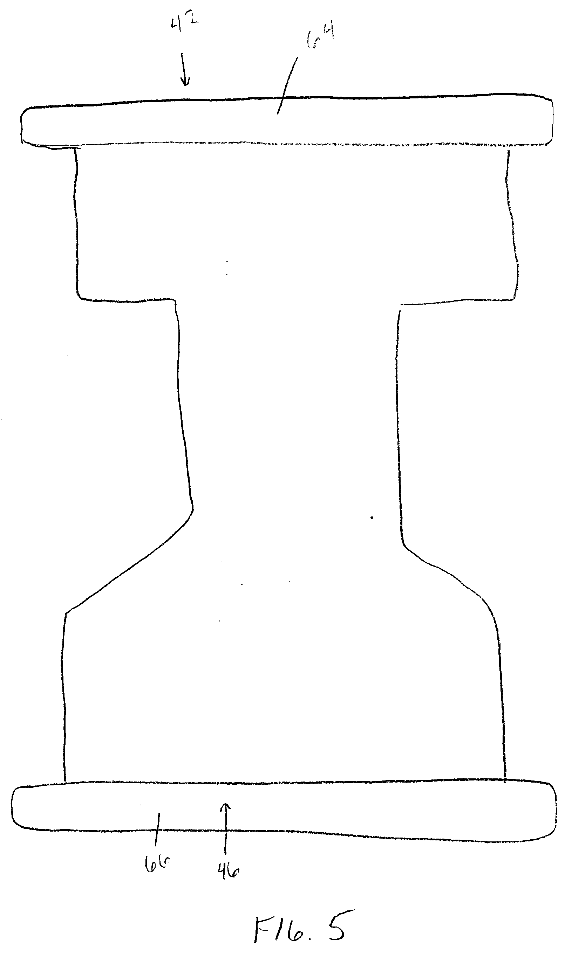

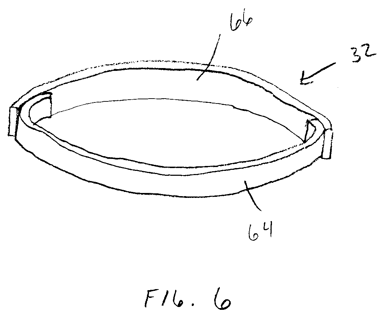

In the embodiment shown in FIGS. 1-3, the front waistband portion 64 has a length dimension greater than the length dimension of the front waistborder section 42. The length dimension of the back waistband portion **66** is equal to the length dimension of the back waistborder section **46**. The length dimension of the back waistband portion **66** may be less than the length dimension of the back waistborder section **46** so long as the total length of the two waistband portions **64** and **66** is greater than the total length of the two waisborder sections **42** and **46**. In the embodiment shown in FIG. 4, the back waistband portion **66** has a length dimension greater than the length dimension of the back waistborder section **46**. In the embodiment of FIG. 5, both the front and back waistband portions, **64**, **66**, have length dimensions greater than the length dimension of the waistborder sections **42** and **46**, respectively, to which they are joined. As shown in FIG. 6, the greater length dimension of at least one of the waistband portions **64**, **66**, permits the waistband to be overlapped creating elasticity about the entire circumference of the waist opening.

The widths of the front and back waisborder sections **42**, **46** substantially correspond to the respective widths of front and back waistband portions **64**, **66**. Length is measured along a line generally parallel to transverse centerline **60** and width is measured along a line generally parallel to longitudinal centerline **62**. A desired width range of waistband portions **64**, **66** is between about 1 centimeter to about 8 centimeters, and a more preferred range is between about 2 centimeters to about 4 centimeters. In the case where, for example, one or both of the waistband portions **64**, **66** have some other geometric shape, i.e., not an elongate rectangular shape as illustrated each waistborder section **42**, **46** desirably would have substantially the same geometric shape as its respective waistband portion **64**, **66**.

Garment **10** also comprises a pair of leg elastic members **72** that are suitably joined, for example, between topsheet **34** and backsheet **36**.

Absorbent structure 28 comprises front absorbent edge 74, back absorbent edge 76, and absorbent side edges 78. Front absorbent edge 74 and back absorbent edge 76 are respectively inboard of front waistband portion 64 and back waistband portion 66. Similarly, absorbent side edges 78 are inboard of respective leg elastic member 72. By "inboard" it is meant that absorbent front edge **74**, for example, is closer to transverse centerline **60** than front waistband portion **64**, and absorbent side edges **78** are closer to longitudinal centerline **62** than leg elastic members **72**.

When garment **10** is properly fitted on the wearer, topsheet **34** faces toward the body of the wearer, and may or may not be the layer that directly contacts the skin. Topsheet **34** can be a liquid permeable, elastic or non-elastic, substantially hydrophobic material, such as a spunbonded web of synthetic polymer filaments. Topsheet **34** can also be a meltblown web or a bonded carded web of synthetic polymer filaments. Suitable synthetic polymers include, for example, polyethylene, polypropylene, and polyesters. Topsheet **34** has a pore size that readily allows the passage therethrough of liquids, such as urine and other body exudates. If desired, topsheet **34** can be treated with surfactants to selectively adjust its degree of wettability, and can also be selectively embossed or perforated with discrete slits or holes extending therethrough. Suitable topsheet materials can have a basis weight between about 10 grams per square meter (gsm) to about 26 gsm, and a thickness between about 0.013 centimeters to about 0.064 centimeters. The thickness of the topsheet material can be determined by employing an Ames Bulk Test (ASTM C-1777) performed at a restraining pressure of 0.2 psi (1.38 kPa).

Backsheet **36**, which may or may not be the outermost layer of garment **10**, can be liquid permeable or liquid impermeable, and may or may not have breathability, i.e., be vapor permeable. A suitable liquid permeable backsheet **36** is a nonwoven bicomponent web having a basis weight between about 15 gsm to about 50 gsm. The nonwoven bicomponent web may be a spunbond bicmoponent web, or a bonded carded bicomponent web. Suitable bicomponent fibers are a wettable, polyethylene/polypropylene bicomponent fiber available from CHISSO Corporation, Osaka, Japan. In this particular bicomponent fiber, the polypropylene forms the core and the polyethylene forms the sheath of the fiber. Other fiber orientations are possible, such as multilobe, side-by-side or end-to-end. Another suitable liquid permeable material is a liquid permeable spundbond polypropylene nonwoven web having a basis weight between about 15 gsm to about 50 gsm.

A suitable liquid impermeable backsheet **36** is a 0.0015 centimeter thick polyethylene film. Backsheet **36** can also be a two-ply laminate, in which the innermost layer can be the above described liquid impermeable film or any other suitable liquid impermeable layer, and the outermost layer can be the above-described liquid permeable spunbond polypropylene nonwoven web or any other suitable liquid permeable layer. Backsheet **36** desirably has a thickness within the range of about 0.0013 to about 0.0051 centimeters.

Absorbent structure 28 can comprise any suitable absorbent material, natural or synthetic, or a combination thereof, along with superabsorbent material. The absorbent material of which absorbent structure 28 is made may also be encased in a tissue wrap (not shown) in order to maintain the integrity of the absorbent material comprising absorbent structure 28.

The construction of garment **10** can be accomplished in any conventional manner well known in the art. For example, the structural elements can be joined together in any manner, such as by heat sealing or ultrasonic bonding, or by adhering the elements together with a suitable adhesive. Suitable adhesives can be obtained from Findley Adhesives, Inc., Wauwatosa, Wis., and can applied in any manner, such as by spraying, slot-coat extrusion, printing, or the like. The applied adhesive can be in any desired configuration, such as continuous or discontinuous beads, continuous or discontinuous swirls, meltblown patterns, spray patterns, or the like.

Front elastic side members **68** can have the same or different geometry from back elastic side members **70**. Each front elastic side member 68 and each back elastic side member 70 comprises an elastic layer sandwiched between topsheet **34** and backsheet **36**. The elastic layer can be any suitable elastic material, one such material being a block copolymer of styrene-ethylbutadiene-styrene. Other types of materials of which elastic layer can be made are the KRATON® G series from The Shell Chemical Company such as KRATON® G-1652, KRATON® GX-1657 and KRATON® G 2740X. The KRATON® D series can also be used, as well as polyester elastomeric materials, polyurethane elastomeric materials, and polyamide elastomeric materials. The elastic layer can be a film, nonwoven web, or ribbons or threads of synthetic or natural rubber arranged, for example, in a spaced, parallel manner.

Each elastic layer is operatively elastically joined to a portion of either topsheet **34** or backsheet **36**, preferably to portions of both topsheet **34** and backsheet **36**, in order to provide elasticity to those portions. One example of providing elastic side sections **20** is described in U.S. Pat. No. 4,940,464, issued Jul. 10, 1990, to inventor Van Gompel et al..

Still other examples of elastic materials and composites are described in U.S. Pat. No. 4,720,415, issued Jan. 19, 1988, to Vander Wielen et al.; U.S. Pat. No. 4,657,802, issued Apr. 14, 1987, to Morman; and U.S. Pat. No. 4,652,487 issued Mar. 24, 1987, to Morman.

Each elastic side section **20** in disposable absorbent garment **10** can have an elasticity between about 50% to about 250%, and more desirably an elasticity between about 100% to about 150%.

Continuous waistband **32** can be an elastomeric, clothlike, nonwoven fibrous material, such as an elastomeric stretch bonded laminate web or an elastomeric meltblown web. By proper selection of materials, continuous waistband **32** can be rendered temporarily elastically inhibited, such as by compression. Once temporarily elastically inhibited, the elastic material, of which waistband **32** is comprised, can be activated, such as by treating with heat, to recover to a state of elasticity.

In one specific embodiment, waistband **32** comprises an elastomeric nonwoven fibrous web that is substantially vapor-permeable. Examples of suitable elastomeric nonwoven fibrous webs are described in U.S. Pat. No. 4,663,220 issued May 5, 1987, to Wisneske et al. Examples of composite fabrics comprising at least one layer of nonwoven textile fabric joined to a fibrous elastic layer are described in European Patent Application EPA 0 217 032 published on Apr. 8, 1987, inventors J. Taylor et al. The composite nonwoven fabrics are commonly referred to as stretch bonded laminates.

In another embodiment, waistband **32** comprises a composite elastomeric web comprising individual, discrete strips or strands of elastomeric material secured to one or more nonwoven fibrous layers. Such a composite elastomeric web may, for example, comprise an elastomeric meltblown material arranged in a selected pattern of strips and suitably sandwiched and joined between two layers of nonwoven fibrous material. This material, as well as other, is described in U.S. Pat. No. 4,861,652 issued Aug. 29, 1989. Still other useful composite elastic materials are described in U.S. Pat. No. 4,883,549, issued Nov. 28, 1989.

Front and back waistborder sections **42**, **46** define continuous waistborder **30**, and have front and back waistband portions **64**, **66** suitably joined thereto, respectively.

Front waistband portion **64** is suitably joined, such as by ultrasonic, heat, or adhesive point bonding, to front waistborder section **42**.

In a similar manner, back waistband portion **66** is joined to back waistborder section **46**. Thereafter, disposable absorbent garment **10** is folded along a fold line generally parallel to transverse centerline **60**, and front section **14** is joined to back section **66** along lap seams **22**.

It will be appreciated that the elasticity about waist opening **24** of garment **10** depends on various factors, such as the types of materials of which waistband **32** and waistborder **30** are comprised, their elastic characteristics, and the like.

While this invention has been described as having a preferred embodiment, it will be understood that it is capable of further modifications. This application is therefore intended to cover any various, equivalents, uses or adaptations of the invention following the general principles thereof, and including such departures from the present disclosure as come or may come within known or customary practice in the art to which this invention pertains and falls within the limits of the appended claims.

**Claims**

1. A disposable garment (10), comprising:

a pant body (12) defining a waist opening (24) and a pair of leg openings (26), said pant body (12) comprising a front section (14), a back section (16), and a crotch section (18);

a continuous waistborder (30) comprising a front waistborder section (42) in said front section (14) and a back waistborder section (46) in said back section (16), said front waistborder section (42) having a length dimension and said back waistborder section (46) having a length dimension;

an elastic waistband (32) comprising a front waistband portion (64) joined to said front waistborder section (42) and a back waistband portion (66) joined to said back waistborder section (46), said front waistband portion (64) having a length dimension and said back waistband portion (66) having a length dimension, characterized by at least one of said elastic waistband portions having a length dimension greater than the length dimension of the respective waistborder section to which said elastic waistband portion is joined.

2. The garment of claim 1 wherein said front waistband portion (64) has a length dimension greater than the length dimension of said front waistborder section (42).

3. The garment of claim 1 wherein said back waistband portion (66) has a length dimension greater than the length dimension of said back waistborder section (46).

4. The garment of claim 1 wherein said back waistband portion (66) has a length dimension greater than the length dimension of said back waistborder section (46) and said front waistband portion (64) has a length dimension greater than the length dimension of said front waistborder section (42).

5. The garment of claim 1 wherein said waistband has a width between about 1 centimeter and about 8 centimeters.

6. The garment of claim 1 wherein said pant body (12) comprises a topsheet (34) and a backsheet (36).

7. The garment of claim 6 further comprising an absorbent structure (28) positioned between said topsheet (34) and said backsheet (36).

8. The garment of claim 7 further comprising elastic leg members (72) positioned between said topsheet and said backsheet.

9. The garment of claim 1 wherein the total length dimension of said front and back waistband portions (64, 66) is greater than the total length dimension of said front and back waistborder sections (42, 46).

FIG. 1

FIG. 2

FIG 3

FIG. 4

FIG. 5

FIG. 6

European Patent
Office

**EUROPEAN SEARCH REPORT**

Application Number

EP 98 10 4437

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | GB 2 294 865 A (MOELNLYCKE AB) 15 May 1996<br>* page 10, line 12 - line 19; claim 8;<br>figure 4 * | 1-9 | A61F13/15 |
| A | WO 94 09736 A (PROCTER & GAMBLE FAR EAST INC ;ROLLAG KEITH W (JP); YAP SHYI EARN) 11 May 1994<br>* abstract; figures * | 1,5-8 | |
| A | EP 0 255 500 A (MOELNLYCKE AB) 3 February 1988<br>* claims; figures * | 1,5-8 | |
| A | US 4 585 447 A (KARAMI HAMZEH) 29 April 1986<br>* claim 1; figures * | 1,6,7 | |
| A | EP 0 638 304 A (PROCTER & GAMBLE) 15 February 1995<br>* column 2, line 26 - line 30; claims 1,2,16 * | 1,5-8 | TECHNICAL FIELDS SEARCHED (Int.Cl.6)<br><br>A61F |

The present search report has been drawn up for all claims

| Place of search | Date of completion of the search | Examiner |
|---|---|---|
| THE HAGUE | 22 July 1998 | Mirza, A |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)